# EUROPEAN PATENT SPECIFICATION

(11) **EP 2 955 217 B1**
(45) Date of publication and mention of the grant of the patent: **20.11.2019**
(21) Application number: 15171491.2
(22) Date of filing: 10.06.2015
(51) Int. Cl.: C10L 5/44, C10L 9/08, C10L 3/08, C12M 1/00, C02F 11/04, C02F 11/06, C02F 11/12, C02F 11/16

(54) **METHOD AND SYSTEM FOR PROCESSING BIOMASS**
VERFAHREN UND VORRICHTUNG ZUR BEHANDLUNG VON BIOMASSE
PROCÉDÉ ET APPAREIL POUR LE TRAITEMENT DE BIOMASSE

(30) Priority: 10.06.2014 BE 201400437
(43) Date of publication of application: 16.12.2015
(73) Proprietor: B.A.T. SERVICES bvba, 9051 Sint-Denijs-Westrem (BE)
(72) Inventor: De Lathauwer, Bart, 9051 Gent (BE)
(74) Representative: Debruyne, Delphine Jenny

(56) References cited:
- WO-A1-2014/057102
- JP-A- H03 154 616
- US-A1- 2011 070 628
- US-A1- 2013 172 636

## Description

### TECHNICAL FIELD

The present invention concerns a method and system for the processing of biomass into biofertiliser, and optionally electricity, particularly green electricity.

### BACKGROUND

The processing of biomass into biofertiliser and/or green energy or bio-energy is known in prior art. Examples of known technologies are fermentation, composting, and incineration or gasification of biomass, such as disclosed in US 2011/070628 A1 or WO 2014/057102 A1.

Biomass, including wet biomass, is converted by fermentation into biogas that can be further incinerated in e.g. a gas engine or gas turbine in order to generate (green) electricity (and heat). The material remaining after fermentation (i.e. the digestate) can be spread on the ground or be treated as fertiliser after further processing such as e.g. drying. The wet digestate is rich in nutrients. Nitrogen (N) that is present in the biomass will, for example, be converted into ammonium (NH₄⁺) during the fermentation process. A portion of the nitrogen, however, will disappear from the digestate, and in drying, by evaporation in the form of ammonia (NH₃). The wet digestate is also impractical for transport.

Composting takes place due to the activity of bacteria and moulds that are naturally present in the organic material (i.e. the biomass). They break the material down and use the products for their own life processes. In this process, nitrogen (N) is converted into nitrate (NO₃⁻), a form of mineral nitrogen that readily leaches out.

The thermal energy released by incineration of biomass can be used as such, e.g. for heating, or be further converted into (green) electricity using an Organic Rankine Cycle (ORC) turbine or steam turbine. The non-combustible fraction of the biomass remains in the incineration facility as ash and can be used as fertiliser. However, this fertiliser is poor in nitrogen (N), which leaves the incineration facility as NOₓ together with the flue gases.

Other nutrients can leave the incineration facility as fly ash with the flue gases, unless these nutrients are collected, e.g. with a dust filter. Incineration is unfavourable from an energy standpoint for the processing of wet biomass, which must first be dried before it is incinerated.

### SUMMARY

The method according to the invention solves one or several of the problems of biomass production associated with the technologies known in prior art by synergistically integrating these known technologies.

In particular, the present invention provides a method for the processing of biomass comprising fermentation, drying, incineration or gasification of the biomass, wherein at least nitrogen (N) is separated from the biomass before the incineration or gasification process and added after incineration or gasification to the ash or the residue respectively, which results in a biofertiliser with increased recovery of nutrients from the biomass. The biofertiliser is further characterised by being dry.

In addition, (green) energy production, particularly the production of (green) electricity, can be increased with the method according to the invention. If the thermal energy released by incineration is used for (green) electricity production, this results in a higher yield than direct incineration of the biomass, as the biomass is dried before incineration. In this way, wet biomass can also be efficiently incinerated while generating green electricity. The yield can be increased further by converting the biogas formed in fermentation of the biomass into electricity and residual heat, and then converting this residual heat, together with the thermal energy from the incineration process, into electricity and residual heat.

The yield can also be increased by gasifying the biomass instead of incinerating it, with the resulting gas fraction being further converted into electricity and residual heat. Optionally, this residual heat, together with the residual heat obtained by converting the biogas into electricity, can further be converted into electricity and residual heat in order to increase the yield even further.

The present invention therefore provides a method for the processing of biomass optionally to produce (green) energy, particularly (green) electricity, and to produce biofertiliser, as defined in the attached claims.

Also provided herein is a system (100) for the processing of biomass by the method as defined in the attached claims.

### DESCRIPTION OF THE FIGURES

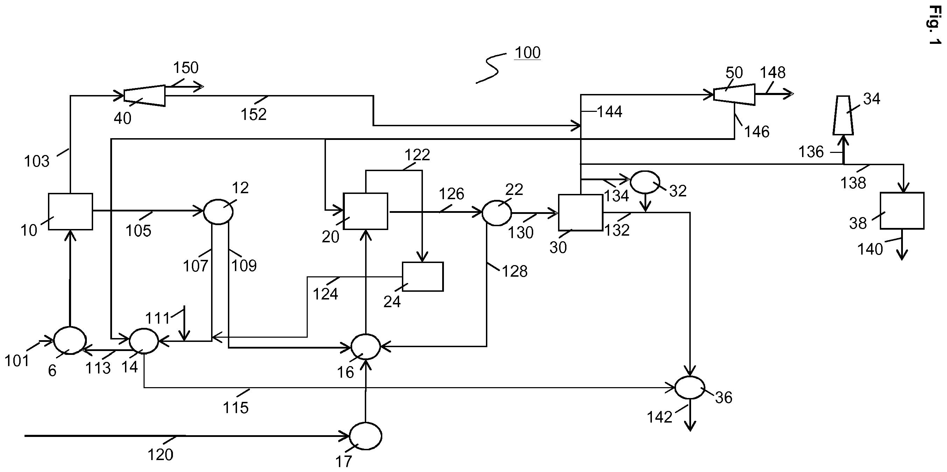

**Fig. 1** shows a schematic depiction of a system (100) and method according to the invention.

### DETAILED DESCRIPTION

Before the present methods and systems according to the invention are described, it must be understood that the invention is not limited to the particular methods and systems described, as such methods and systems can naturally vary. It must also be clear that the terminology used herein is not intended to be limiting, as the scope of the present invention can only be limited by the relevant claims.

Unless otherwise defined, all technical and scientific terms used herein have the same meaning as is generally understood by a person skilled in the art in the field to which this invention belongs. Although all methods and materials that are the same or equivalent to those described herein may be used in practice or in testing of the present invention, the preferred methods and materials will now be described.

In this description and in the attached claims, the singular forms "a," "the" [neuter definite article]" and "the" [non-neuter definite article] include the plural forms, unless the context clearly indicates otherwise.

The terms "comprising", "comprises" and "comprise" as used herein are synonyms for "including" or "containing", "contains" and "contain" and do not exclude any open-ended or closed-ended additional unspecified members, elements or method steps.

The terms "comprising", "comprises" and "comprise" also include the term "consisting of".

The present invention provides a method for the processing of biomass, for the production of biofertiliser, particularly a dry biofertiliser, and optionally for the production of (green) energy, particularly (green) electricity, comprising the following steps:
a) fermentation of a first portion of the biomass into biogas and digestate;
b) separation of the digestate obtained in step a) into a liquid fraction and a solid fraction;
c) separation of nitrogen (N), from the liquid fraction of the digestate obtained in step b) and addition of said nitrogen to the residue obtained after gasification in step e) or the ash obtained in step e);
d) drying of the solid fraction of the digestate obtained in step b); and
e) incineration of the dried solid fraction of the digestate obtained in step d) to ash, or gasification of the dried solid fraction of the digestate obtained in step d); and optionally:
f) conversion of the biogas obtained in step a) into electricity and residual heat;
   and
g) conversion of the heat released in the incineration of step e) and the residual heat obtained in step f) into electricity and residual heat, or conversion of the gas fraction after gasification in step e) into electricity and residual heat.

The method according to the invention can be a linear or non-cyclical method. The terms "linear" or "non-cyclical" as used herein mean that end products of the method, such as the nutrient-enriched residue from the gasification process or the biofertiliser, are not again subjected to one or more steps of the method.

Therefore, preferred embodiments of the present invention are methods wherein the nitrogen-enriched ash of the gasification residue obtained in step e) is not subjected to one or more steps of the method such as fermentation and/or incineration.

Nitrogen (N) present in the biomass, particularly the first portion of the biomass, is converted during fermentation into ammonium (NH₄⁺), which is recovered before incineration or gasification and then added to the ash obtained in step e) or the residue obtained after gasification in step e). In this way, more nitrogen can be recovered from the biomass in the biofertiliser compared to incineration or gasification alone. In addition, the nitrogen in the biofertiliser is composed of ammonium sulphate ((NH₄)₂SO₄), which leaches out less than, for example, nitrate (NO₃⁻) formed during composting.

In further embodiments, nutrients, preferably nitrogen (N), are separated from the vapour released in the drying of step d) and added to the ash obtained in step e) or the residue obtained after gasification in step e). This recovery of nutrients will result in a further increase in the percentage of nutrients from the biomass in the biofertiliser.

In some embodiments, the method further comprises the following step: h) conversion of the residual heat obtained in step f) and the residual heat obtained in step g) into electricity and residual heat.

This type of integrated method allows the synergistic production of more electricity, particularly green electricity, from the biomass than in the separate processes.

The term "biomass" as used herein refers to the biodegradable fraction of products, waste materials and residues from agriculture (including waste streams), forestry and related industries, as well as the biodegradable fraction of industrial and household waste.

In a preferred embodiment, secondary biomass is processed. The term "secondary biomass" as used herein refers to biomass that cannot be used for human consumption.

The various process steps are described in greater detail below. The various process steps should preferably take place at one site, but methods in which one or more process steps take place at another site are also provided herein.

### Fermentation or anaerobic digestion

The terms "fermentation", "fermenting" and "anaerobic digestion" are used herein as synonyms and refer to a microbial process that takes place in the absence of oxygen (i.e. anaerobically) in which biomass is converted into simpler products such as methane (CH₄), carbon dioxide (CO₂), H₂ and H₂O.

Fermentation typically occurs at temperatures of between 15 °C and 60 °C, depending on the microorganisms involved. The microorganisms used in a fermentation process are typically a combination of various kinds of microorganisms such as thermophilic or mesophilic bacteria.

Another parameter in the fermentation process is the ammonia concentration in the reactor. Preferably, the ammonia concentration in the reactor is less than 5 g/L. In order to obtain an optimal ammonia concentration in the reactor, the biomass, particularly the first portion of the biomass, is diluted with water, e.g. water after condensation of the water vapour released by evaporation of the liquid fraction of the digestate.

Fermentation is typically carried out in a fermentation unit or fermentation facility (10). These may take various forms, such as a concrete or metal tank reactor or an oval reactor. A further distinction can be made between continuously fed, semi-continuously fed, and batch reactors. In continuously fed reactors, mixing of fresh and old material in the fermentation unit is necessary so that the microorganisms come into contact with the freshly-fed material more quickly. Mixing can be carried out e.g. by stirring, circulation of the liquid, or by feeding in biogas.

The wet end product of fermentation is referred to as the "digestate". The composition of the digestate primarily depends on the composition of the biomass that is fermented.

In addition, biogas is also produced in the fermentation facility. The term "biogas" is understood herein to refer to the gas mixture produced after fermentation of the biomass, particularly the first portion of the biomass. The biogas primarily comprises methane (CH₄) and carbon dioxide (CO₂), and can further comprise one or more components selected from the group composed of H₂O, H₂S, and NH₃. The exact composition of the biogas is determined by the composition of the biomass that is fermented.

In some embodiments of the method according to the invention, the biomass, particularly the first portion of the biomass, is pre-treated before fermentation. Possible pre-treatment techniques include size reduction, dry separation, wet separation, pasteurisation, sterilisation, acidification, etc. Usually, pre-treatment consists of a combination of two or more of the listed techniques.

The biomass that is fermented in the method according to the invention is referred to as a first portion of the biomass. The first portion of the biomass may be either wet (i.e. liquid) or dry (i.e. solid). Preferably, the first portion of the biomass comprises nitrogen-rich biomass, i.e. biomass with a nitrogen content of between 1% and 20%, preferably between 2% and 20%, and even more preferably between 5% and 20%, based on the total weight of the biomass. Non-limiting examples of biomass that can be used as the first portion of the biomass in the method according to the invention are product streams such as corn, glycerine, soapstock, etc.; waste streams such as fat, flotate, feed, fine green, etc.; and fertiliser streams from e.g. chickens, pigs and cattle.

### Separation of the digestate

In a separating unit (12), the solid and liquid fractions of the digestate are separated from each other. Examples of a separating unit (12) are a centrifuge or a screw press. A centrifuge is preferably used.

The difficulty of dewatering the digestate will vary depending on the biomass being fermented and the fermentation process itself. High-fibre material can sharply increase dewaterability. Chemicals such as polyelectrolytes or polymers may also be used for better dehydration.

The term "high-fibre solid or thick fraction of the digestate" is understood herein to refer to the fraction of the digestate after separating that contains at least 15% (w/w) of dry matter.

The term "liquid or thick fraction of the digestate" is understood herein to refer to the fraction of the digestate after separating that comprises at most 5% (w/w) dry matter.

Not all nutrients are distributed in the same manner between the two fractions after separation: phosphorus largely goes to the solid fraction and nitrogen can mostly be found in the liquid fraction.

In some embodiments, the method according to the invention may further comprise a step of sanitising the digestate before or after separation.

### Conversion of biogas into electricity and residual heat

The biogas can further be converted into electricity, heat, mechanical energy or a combination of two or more thereof. In addition, it is also possible, optionally after purifying the biogas into biomethane, to impart value to it as a renewable-source natural gas. The bio-natural gas can e.g. be injected into the natural gas network, or it can be compressed at a pressure of 200 to 250 bar into bio-CNG (compressed natural gas), more precisely referred to as CBG (compressed biogas) as well as CBM (compressed biomethane). Biomethane, preferably purified biomethane, can also be used as a precursor for the production of biopolymers.

In one embodiment, the biogas is converted into electricity and residual heat. As the electricity is derived from biomass, it can be referred to as "green electricity". In some embodiments, the biogas is converted into electricity and residual heat using a gas turbine. The biogas is incinerated in the incineration chamber of a gas turbine. After this, the turbine activates a generator, thus producing energy. In some embodiments, the biogas is converted into electricity and residual heat using a gas engine. Biogas can also be converted into electricity and residual heat using a fuel cell.

### Recovery of nutrients from the liquid fraction of the digestate

In the methods according to the invention, nutrients, particularly nitrogen (N), phosphorus (P) and potassium (K) are recovered to the maximum extent from the biomass. For this purpose, at least nitrogen is separated from the liquid fraction of the digestate. They can also be used as is as fertiliser, but are preferably mixed with the ash derived from incineration of the residue of gasification in step e) of the method according to the invention.

In some embodiments, nutrients are separated from the liquid fraction of the digestate by evaporation. The non-volatile nutrients present in the liquid fraction are concentrated by evaporating the water. The residual heat is preferably derived from step g) of the method according to the invention used for this evaporation process.

For separating nitrogen (N) from the liquid fraction by evaporation, a strongly acidic solution, particularly a sulphuric acid solution, can be added to the liquid fraction before evaporation. Specifically, nitrogen (N) is converted to volatile ammonia (NH₃) in the fermentation process, which then disappears in the vapour phase on evaporation of the liquid fraction. Addition of the sulphuric acid solution (H₂SO₄) causes ammonium sulphate (NH₄SO₄) to be formed, which remains as a residue together with the other non-volatile nutrients after evaporation. As an alternative, the sulphuric acid (H₂SO₄) can be added to the vapour phase after evaporation (i.e. acidic water or gas scrubbing) in order to produce ammonium sulphate (NH₄SO₄).

An alternative technology for separating nitrogen from the liquid fraction of the digestate is steam stripping, in which steam is blown through the liquid fraction so that volatile nutrients (e.g. ammonia (NH₃)) enter the gas phase. After cleaning of the gas phase, e.g. using an acidic gas or air scrubber, with sulphuric acid (H₂SO₄), nitrogen (N) can therefore be separated in the form of ammonium sulphate (NH₄SO₄) from the liquid fraction of the digestate.

### Drying

The solid fraction of the digestate is dried in the methods according to the invention.

In one embodiment, the residual heat derived from step g) of the method according to the invention is used for the drying process.

In other embodiments, drying is carried out biothermally. The term "biothermal drying" is understood herein to mean that the heat for the drying process is biological heat, i.e. heat derived from microorganisms that break down the biomass in the presence of oxygen.

In a preferred embodiment, biothermal drying is carried out for a maximum period of 10 days. In a more preferred embodiment, biothermal drying is carried out for a maximum period of 8 days. In a most preferred embodiment of the invention, biothermal drying is carried out for a maximum period of 7 days (i.e. a maximum of 1 week).

Microorganisms are naturally present in biomass. The digestate, however, primarily comprises anaerobic microorganisms from the fermentation process. These anaerobic microorganisms should not grow under the aerobic conditions of drying. For this reason, a second portion of the biomass must be mixed with the solid fraction of the digestate before the entire mixture can be biothermally dried. This second portion of the biomass preferably comprises biomass with a dry matter content of between 25% and 100%, e.g. between 25% and 50%, based on the total weight of the biomass. Preferably, this second portion of the biomass is nitrogen-poor, i.e. biomass with a nitrogen content of less than 2%, and preferably less than 1%, e.g. about 0.5%, based on the total weight of the biomass. More preferably, the nitrogen (N) is present in the second portion of the biomass in the form of ammonia compounds. Non-limiting examples of biomass that can be used as the second portion of the biomass are waste streams such as pruning waste, wood waste, coarse green, spent mushroom compost, etc. and fertiliser streams originating from e.g. chickens, pigs, and cattle.

In some embodiments of the method according to the invention, the second portion of the biomass is pre-treated, e.g. reduced in size or crushed, before mixing with the solid fraction of the digestate.

In still other embodiments, drying is carried out biothermally and using the residual heat derived from step g) of the method according to the invention. In this embodiment as well, the solid fraction of the digestate must first be mixed with a second portion of the biomass before drying.

The drying process typically occurs in a drying unit or drying facility (20). Any type of drying unit may be used, such as a tunnel dryer, fluidised-bed dryer, tumble-dryer, etc.

One should preferably use a tunnel dryer, with which drying can be carried out both biothermally and using (residual) heat.

### Recovery of volatile nutrients from the drying process

In drying the solid fraction of the digestate, optionally mixed with a second portion of the biomass, nitrogen (N) in the form of ammonia (NH₃), will disappear on evaporation.

In some embodiments, nutrients, preferably nitrogen (N), are separated from the vapour released in the drying of step d). Preferably, these nutrients are then added to the ash obtained in step e) of the method according to the invention or the residue obtained after gasification in step e) of the method.

Separation of nutrients, preferably nitrogen (N), from the vapour released in the drying process can be carried out by gas cleaning, wherein the vapour is fed through a gas scrubber (24) with a liquid (i.e. the wash water). Before separation of ammonia (NH₃) from the vapour, one should preferably use an acidic gas scrubber comprising a sulphuric acid solution, which separates the ammonia (NH₃) from the vapour via a chemical reaction into ammonium sulphate (NH₄SO₄).

The nutrients can then be separated from the wash with technologies such as those described above for the separation of nutrients from the liquid fraction of the digestate.

The wash water can also be mixed with the liquid fraction of the digestate obtained in step b) of the method according to the invention, and the nutrients can be separated therefrom as described above.

### Separation of the solid fraction of the digestate

In some embodiments, the dried solid fraction of the digestate, optionally mixed with the dried second portion of the biomass, is separated by size in a separating unit (22), e.g. a sieve. The finest fraction (i.e. a fraction with a (grain) size of at most 20 mm) can then leave the system described herein, while the remaining coarser fraction (i.e. a fraction with a (grain) size of at least 20 mm) is subjected to further steps of the methods described herein, such as incineration or gasification. In further embodiments, the coarser fraction can be further separated into a fraction with a (grain) size of at least 50 mm and a fraction with a (grain) size of at most 50 mm, preferably a (grain) size of between 20 mm and 50 mm. The fraction with a (grain) size of at most 50 mm, and preferably a (grain) size of between 50 mm and 100 mm, can then be mixed with the solid fraction of the digestate and optionally a second portion of the biomass for the purpose of further drying. The fraction with a (grain) size of at most 50 mm, and preferably a (grain) size of between 20 mm and 50 mm, can then be incinerated or gasified.

### Incineration

In some embodiments, in step e) of the method according to the invention, the dried solid fraction of the digestate, and optionally the dried second portion of the biomass, preferably the fraction thereof with a (grain) size of at least 20 mm, and even more preferably the fraction thereof with a (grain) size of between 20 mm and 50 mm, is incinerated to ash, flue gases comprising CO₂ and H₂O, and thermal energy in an incineration unit (30). The ashes comprise the non-combustible fraction of the dried solid fraction of the digestate, optionally mixed with the dried second portion of the biomass, and can be used as fertiliser. As the ashes are completely derived from biomass, the fertiliser can be referred to as "biofertiliser".

The ash typically comprises bottom ash, which remains in the incineration unit (30), and fly ash, which disappears together with the flue gases. In some embodiments, the fly ash is collected and added to the bottom ash. By this method, the recovery of nutrients from the biomass can be further increased. Collection of the fly ash can be carried out with a dust filter (32).

Because at least nitrogen (N), which is separated from the liquid fraction of the digestate and possibly also from the vapour released in drying, is added to the ash, particularly the bottom ash, a biofertiliser is obtained with increased recovery of nutrients. In some embodiments, at least 80%, e.g. at least 85%, and preferably at least 90%, e.g. at least 91%, 92%, 93%, 94%, or 95%, of the nutrients from the biomass are recovered in the biofertiliser.

The (nutrient-rich) biofertiliser is further characterised by being dry. This provides a considerable number of practical advantages, e.g. making the fertiliser much easier to transport compared to (nutrient-enriched) wet digestate, for example.

Preferably, the biofertiliser is also free of microorganisms such as algae or bacteria. In some embodiments, the flue gases are cleaned, e.g. by gas cleaning. This limits emissions of harmful substances into the environment. In some embodiments, the dried solid fraction of the digestate, optionally mixed with the dried second portion of the biomass, can be mixed with a third portion of the biomass before incineration. This third portion of the biomass is preferably combustible biomass. Non-limiting examples of combustible biomass are product streams such as straw, wood dust, etc. and waste streams such as wood and sieve overflow.

### Gasification

In some embodiments, in step e) of the method according to the invention, the dried solid fraction of the digestate, and optionally the dried second portion of the biomass, preferably the fraction thereof with a (grain) size of at least 20 mm, and even more preferably the fraction thereof with a (grain) size of between 20 mm and 50 mm, is gasified in a gasification unit.

The term "gasification" of biomass is understood herein to refer to a thermal process wherein at least a portion of the biomass is converted into a gaseous phase (or syngas) by thermal cracking.

The residue that remains after gasification of the biomass can be used as fertiliser. As the residue is completely derived from biomass, the fertiliser can be referred to as "biofertiliser".

The biofertiliser described herein is characterised by increased recovery of nutrients because at least nitrogen (N), from the liquid fraction of the digestate and possibly also from the vapour released in drying, is separated and added to the gasification residue. The (nutrient-rich) biofertiliser is further characterised by being dry. Preferably, the biofertiliser is also free of microorganisms such as algae and bacteria.

### Conversion of the gas fraction obtained after gasification into electricity and residual heat

The gas fraction obtained after gasification of the dried solid fraction of the digestate, and optionally the dried second portion of the biomass, can be incinerated, with recovery of energy (electricity and residual heat), or can be used in the chemical industry as a raw material.

In some embodiments, the gas fraction obtained after gasification is converted into green electricity and residual heat. In this way, more green electricity can be obtained with the method according to the invention than when the dried solid fraction of the digestate is incinerated.

Recovery of energy from syngas can be carried out, for example, by means of a classical steam cycle, or by feeding to an engine, gas turbine or CCGT. In some embodiments, the gas fraction obtained after gasification is converted into green electricity and residual heat using a gas engine.

### Conversion of the thermal energy into electricity and residual heat

The thermal energy released by incineration process in step e) of the method according to the invention can be used as such, e.g. for the heat-requiring processes of the method according to the invention, such as the drying process and evaporation of the liquid fraction of the digestate.

In some embodiments, the thermal energy is converted into electricity and residual heat. A cycle referred to as the Rankine Cycle is generally used for this purpose. In this cycle, water is heated to steam. The steam then expands through a steam turbine that provides work to the turbine. The turbine then activates the electricity generator. When the steam in the Rankine Cycle is collected using an organic fluid such as pentane, hexane, toluene, ammonia, etc., this is referred to as the Organic Rankine Cycle (ORC). The advantage of these media is that they evaporate at lower temperatures than steam. Therefore, in some embodiments, the thermal energy is converted into electricity and residual heat using a steam turbine or an ORC-turbine, preferably an ORC-turbine.

As the biomass is dried before the incineration process, the yield of (green) electricity production therefrom is higher than it would be if the biomass were directly incinerated (i.e. without being dried first).

In preferred embodiments of the method according to the invention, the residual heat is obtained by converting biogas into electricity and converting the thermal energy released in the incineration of step e) into electricity and residual heat. In this way, the yield of electricity production from the biomass can be further increased.

In some embodiments, the residual heat is obtained by converting the biogas into electricity, and by converting the residual heat obtained from conversion of the gas fraction obtained after gasification in step e) into electricity and residual heat.

### Conversion of CO₂ into biomass

In some embodiments, the CO₂ released by incineration in step e), and optionally by incineration of biogas, and/or the gas fraction obtained after gasification for conversion into electricity and residual heat, are converted into biomass. Algae and bacteria reproduce very rapidly, and require for this only sunlight, CO₂ and nutrients. The algae biomass and/or bacterial biomass thus obtained can be used as a biofuel or as nutrical (e.g. *Dunaliella salina* due to its high beta-carotene content), or for other applications. In further preferred embodiments, the biomass produced is not further processed according to the method described herein.

In other embodiments, the method described herein is not connected with the production of biomass such as algae biomass.

The ash, particularly the bottom ash, obtained after incineration or the residue obtained after gasification according to the method described herein can be used as a biofertiliser.

The biofertiliser comprises the ash, particularly the bottom ash, obtained after incineration, or the residue obtained after gasification according to the method described herein. The biofertiliser is nutrient-rich, particularly nitrogen-rich, and dry.

Another aspect of the present invention is a system (100) for the processing of biomass, for the production of biofertiliser, and optionally for the production of (green) energy, particularly (green) electricity, comprising:
- a fermentation unit (10);
- a separating unit (12) for separating the digestate into a solid and a liquid fraction;
- a unit (14) for separating nitrogen (N), from the liquid fraction of the digestate, preferably an evaporation unit or a steam stripper;
- a drying unit (20); and
- an incineration unit (30) or gasification unit.

In some embodiments, the system further comprises:
- a unit (40) for converting biogas into electricity and residual heat; and
- a unit (50) for converting the residual heat obtained in conversion of biogas into electricity, and the thermal energy obtained in incineration, into electricity and residual heat.

Alternatively, the system may further comprise:
- a unit (40) for converting biogas into electricity and residual heat;
- a unit for converting the gas fraction obtained in gasification into electricity and residual heat; and optionally
- a unit (50) for converting the residual heat obtained in conversion of biogas into electricity, and the residual heat obtained in conversion of the gas fraction obtained in gasification into electricity, into electricity and residual heat.

### EXAMPLES

### Example 1

An embodiment of the invention is discussed with reference to Fig. 1. A first portion of the biomass (101) is mixed with water (113) originating from the evaporation unit (14) in a mixing unit (6). The mixture is fermented in a fermentation unit (10) into biogas (103) and digestate (105). The wet digestate (105) is separated in a separating unit (12) into a liquid

The biogas (103) produced during the fermentation is fed into a gas turbine (40) or gas engine for the production of electricity (150) and residual heat (152). The residual heat (152), together with the thermal energy (144) from the incineration process, is converted into electricity (148) and residual heat (146) using a steam turbine (50). The residual heat (146) is utilised in the evaporation unit (14) and the tunnel dryer (20).

An example of a mass balance (tons/year) for the various processes of the method described above is shown in Table 1. According to this example, 130,000 tons of biomass annually are converted into biofertiliser containing 93% of the nutrients of the biomass, particularly N, P, K, Ca and Mg, yielding 80,000 MWh of green electricity.

| Fraction composition | First portion of biomass (101) | Solid digestate fraction (109) | Liquid digestate fraction (107) | Evaporated liquid digestate fraction and wash water (115) | Second portion of biomass (120) | Mixture comprising solid fraction digestate and 2nd portion of biomass (109+120) | Dried mixture (126) | Dried fine fraction mixture (130) | Ash (132+ 134) | Biofertiliser (142) | Nutrients recovered (%) |
|---|---|---|---|---|---|---|---|---|---|---|---|
| TOTAL | 50000 | 20000 | 45000 | 10000 | 80000 | 100000 | 60000 | 50000 | 17500 | 20000 | |
| Water | 25000 | 15000 | 42500 | 7500 | 40000 | 55000 | 15000 | 5000 | 0 | 0 | |
| Dry matter | 25000 | 5000 | 1500 | 2500 | 40000 | 45000 | 45000 | 45000 | 17500 | 20000 | |
| Organic matter | 20000 | 2500 | 250 | 250 | 30000 | 32500 | 30000 | 30000 | 0 | 250 | |
| N | 1000 | 250 | 750 | 1000 | 500 | 750 | 500 | (500) | 0 | 1000 | 66.67% |
| P | 500 | 450 | 50 | 50 | 500 | 950 | 950 | 950 | 950 | 1000 | 100% |
| K | 500 | 250 | 250 | 250 | 500 | 750 | 750 | 750 | 750 | 1000 | 100% |
| Ca | 500 | | | | 500 | | | | 1000 | 1000 | 100% |
| Mg | 50 | | | | 50 | | | | 100 | 100 | 100% |
| | | | | | | | | | | GEM | 93% |

## Claims

1. Method for the processing of biomass comprising the following steps:
a) fermentation of a first portion of the biomass into biogas and digestate;
b) separation of the digestate obtained in step a) into a liquid fraction and a solid fraction;
c) separation of nitrogen (N) from the liquid fraction of the digestate obtained in step b) and addition of said nitrogen to the ash obtained in step e) or to the residue obtained after gasification in step e);
d) drying of the solid fraction of the digestate obtained in step b); and
e) incineration of the dried solid fraction of the digestate obtained in step d) to ash or gasification of the dried solid fraction of the digestate obtained in step d); and optionally:
f) conversion of the biogas obtained in step a) into electricity and residual heat; and
g) conversion of the heat released in the incineration of step e) and the residual heat obtained in step f) into electricity and residual heat, or conversion of the gas fraction obtained after gasification in step e) into electricity and residual heat.

2. Method according to claim 1, wherein the ash obtained after incineration in step e) and enriched with the nitrogen, or the residue obtained after gasification in step e) and enriched with the nitrogen, is not again subjected to one or more of steps a) to g).

3. Method according to claim 1 or 2, wherein nitrogen (N) is separated from the vapour released in the drying of step d) and added to the ash obtained in step e) or to the residue obtained after gasification in step e).

4. Method according to any one of claims 1 to 3, wherein the nitrogen from the liquid fraction of the digestate are separated by evaporation, preferably evaporation using the residual heat obtained in step g).

5. Method according to claim 3, wherein the nitrogen from the vapour released in the drying of step d) are separated by gas cleaning of said vapour with wash water, preferably a sulphuric acid solution, and mixing of the wash water with the liquid fraction of the digestate obtained in step b).

6. Method according to any one of claims 1 to 5, wherein in step d), a second portion of the biomass is mixed with the digestate before drying, and wherein the heat for drying is derived from microorganisms that break down the biomass in the presence of oxygen.

7. Method according to any one of claims 1 to 6, wherein the drying in step d) takes place using the residual heat obtained in step g).

8. Method according to any one of claims 1 to 7, wherein the fly ash is collected and added to the bottom ash.

9. Method according to any one of claims 1 to 8, further comprising the step h), conversion of the CO₂ released in the incineration of step e) into biomass, preferably algae biomass and/or bacterial biomass.

10. System (100) for the processing of biomass by the method according to any one of claims 1 to 9, comprising:
- a fermentation unit (10);
- a separating unit (12) for separating the digestate into a solid and a liquid fraction;
- a unit (14) for separating nitrogen (N) from the liquid fraction of the digestate, preferably an evaporation unit or a steam stripper;
- a drying unit (20); and
- an incineration unit (30) or gasification unit.

11. System according to claim 10, further comprising:
- a unit (40) for converting biogas into electricity and residual heat; and
- a unit (50) for converting the residual heat obtained in conversion of biogas into electricity, and the thermal energy obtained in incineration into electricity and residual heat.

## Patentansprüche

1. Verfahren zur Verarbeitung von Biomasse, umfassend folgende Schritte:
a) Fermentation eines ersten Teils der Biomasse zu Biogas und Gärrückstand;
b) Auftrennen des bei Schritt a) erhaltenen Gärrückstands in eine flüssige Fraktion und eine feste Fraktion;
c) Abtrennen von Stickstoff (N) aus der bei Schritt b) erhaltenen flüssigen Fraktion des Gärrückstands und Zugeben des Stickstoffs zu der bei Schritt e) erhaltenen Asche oder zu dem nach der Vergasung bei Schritt e) erhaltenen Rückstand;
d) Trocknen der bei Schritt b) erhaltenen festen Fraktion des Gärrückstands; und
e) Verbrennen der bei Schritt d) erhaltenen getrockneten festen Fraktion des Gärrückstands zu Asche oder Vergasen der bei Schritt d) erhaltenen getrockneten festen Fraktion des Gärrückstands; und gegebenenfalls:
f) Umwandeln des bei Schritt a) erhaltenen Biogases zu Elektrizität und Restwärme; und
g) Umwandeln der bei der Verbrennung bei Schritt e) freigesetzten Wärme und der bei Schritt f) erhaltenen Restwärme zu Elektrizität und Restwärme oder Umwandeln der nach der Vergasung bei Schritt e) erhaltenen Gasfraktion zu Elektrizität und Restwärme.

2. Verfahren gemäß Anspruch 1, wobei die nach der Verbrennung bei Schritt e) erhaltene und mit dem Stickstoff angereicherte Asche oder der nach der Vergasung bei Schritt e) erhaltene und mit dem Stickstoff angereicherte Rückstand nicht wieder einem oder mehreren der Schritte a) bis g) unterworfen wird.

3. Verfahren gemäß Anspruch 1 oder 2, wobei Stickstoff (N) aus dem bei dem Trocknen bei Schritt d) freigesetzten Dampf abgetrennt wird und zu der bei Schritt e) erhaltenen Asche oder zu dem nach der Vergasung bei Schritt e) erhaltenen Rückstand zugegeben wird.

4. Verfahren gemäß einem der Ansprüche 1 bis 3, wobei der Stickstoff aus der flüssigen Fraktion des Gärrückstands durch Verdampfung abgetrennt wird, vorzugsweise Verdampfung unter Verwendung der bei Schritt g) erhaltenen Restwärme.

5. Verfahren gemäß Anspruch 3, wobei der Stickstoff aus dem bei dem Trocknen bei Schritt d) freigesetzten Dampf durch Gasreinigung des Dampfs mit Waschwasser, vorzugsweise einer Schwefelsäurelösung, und Mischen des Waschwassers mit der flüssigen Fraktion des bei Schritt b) erhaltenen Gärrückstands abgetrennt wird.

6. Verfahren gemäß einem der Ansprüche 1 bis 5, wobei bei Schritt d) ein zweiter Teil der Biomasse mit dem Gärrückstand vor dem Trocknen gemischt wird und wobei die Wärme zum Trocknen von Mikroorganismen stammt, die die Biomasse in Gegenwart von Sauerstoff abbauen.

7. Verfahren gemäß einem der Ansprüche 1 bis 6, wobei das Trocknen bei Schritt d) unter Verwendung der bei Schritt g) erhaltenen Restwärme erfolgt.

8. Verfahren gemäß einem der Ansprüche 1 bis 7, wobei die Flugasche gesammelt und zu der Bodenasche zugegeben wird.

9. Verfahren gemäß einem der Ansprüche 1 bis 8, ferner umfassend den Schritt h) der Umwandlung des bei der Verbrennung bei Schritt e) freigesetzten CO₂ zu Biomasse, vorzugsweise Algen-Biomasse und/oder bakterielle Biomasse.

10. System (100) zur Verarbeitung von Biomasse durch das Verfahren gemäß einem der Ansprüche 1 bis 9, umfassend:
- eine Fermentationseinheit (10);
- eine Trenneinheit (12) zum Trennen des Gärrückstands in eine feste und eine flüssige Fraktion;
- eine Einheit (14) zum Abtrennen von Stickstoff (N) aus der flüssigen Fraktion des Gärrückstands, vorzugsweise eine Verdampfungseinheit oder ein Dampfstripper;
- eine Trocknungseinheit (20); und
- eine Verbrennungseinheit (30) oder Vergasungseinheit.

11. System gemäß Anspruch 10, ferner umfassend:
- eine Einheit (40) zum Umwandeln von Biogas zu Elektrizität und Restwärme; und
- eine Einheit (50) zum Umwandeln der bei der Umwandlung von Biogas erhaltenen Restwärme zu Elektrizität und der bei der Verbrennung erhaltenen Wärmeenergie zu Elektrizität und Restwärme.

## Revendications

1. Méthode de transformation de biomasse, comprenant les étapes suivantes:
a) la fermentation d'une première portion de la biomasse en biogaz et digestat;
b) la séparation du digestat obtenu dans l'étape a) en une fraction liquide et une fraction solide;
c) la séparation d'azote (N) à partir de la fraction liquide du digestat obtenu dans l'étape b) et l'addition dudit azote aux cendres obtenues dans l'étape e) ou au résidu obtenu après gazéification dans l'étape e);
d) le séchage de la fraction solide du digestat obtenu dans l'étape b); et
e) l'incinération de la fraction solide séchée du digestat obtenu dans l'étape d) en cendres ou la gazéification de la fraction solide séchée du digestat obtenu dans l'étape d); et éventuellement
f) la conversion du biogaz obtenu dans l'étape a) en électricité et en chaleur résiduelle; et
g) la conversion de la chaleur libérée dans l'incinération de l'étape e) et de la chaleur résiduelle obtenue dans l'étape f) en électricité et en chaleur résiduelle, ou la conversion de la fraction gazeuse obtenue après gazéification dans l'étape e) en électricité et en chaleur résiduelle.

2. Méthode selon la revendication 1, dans laquelle les cendres obtenues après incinération dans l'étape e) et enrichies par l'azote, ou le résidu obtenu après gazéification dans l'étape e) et enrichi par l'azote, ne sont pas soumises de nouveau à une ou plusieurs parmi les étapes a) à g).

3. Méthode selon la revendication 1 ou 2, dans laquelle l'azote (N) est séparé de la vapeur libérée dans le séchage de l'étape d) et ajouté aux cendres obtenues dans l'étape e) ou au résidu obtenu après gazéification dans l'étape e).

4. Méthode selon l'une quelconque des revendications 1 à 3, dans laquelle l'azote issu de la fraction liquide du digestat est séparé par évaporation, préférablement évaporation en utilisant la chaleur résiduelle obtenue dans l'étape g).

5. Méthode selon la revendication 3, dans laquelle l'azote issu de la vapeur libérée dans le séchage de l'étape d) est séparé par un nettoyage de gaz de ladite vapeur par de l'eau de lavage, préférablement une solution d'acide sulfurique, et le mélange de l'eau de lavage avec la fraction liquide du digestat obtenue dans l'étape b).

6. Méthode selon l'une quelconque des revendications 1 à 5, dans laquelle, dans l'étape d), une deuxième portion de la biomasse est mélangée avec le digestat avant séchage, et où la chaleur pour le séchage est dérivée de microorganismes qui décomposent la biomasse en présence d'oxygène.

7. Méthode selon l'une quelconque des revendications 1 à 6, dans laquelle le séchage dans l'étape d) a lieu en utilisant la chaleur résiduelle obtenue dans l'étape g).

8. Méthode selon l'une quelconque des revendications 1 à 7, dans laquelle les cendres volantes sont collectées et ajoutées aux cendres résiduelles.

9. Méthode selon l'une quelconque des revendications 1 à 8, comprenant en outre l'étape h), la conversion du CO₂ libéré dans l'incinération de l'étape e) en biomasse, préférablement une biomasse algale et/ou une biomasse bactérienne.

10. Système (100) de transformation de biomasse par la méthode selon l'une quelconque des revendications 1 à 9, comprenant:
- une unité de fermentation (10);
- une unité de séparation (12) pour séparer le digestat en une fraction liquide et une fraction solide;
- une unité (14) pour séparer l'azote (N) à partir de la fraction liquide du digestat, préférablement une unité d'évaporation ou un distillateur à vapeur;
- une unité de séchage (20); et
- une unité d'incinération (30) ou une unité de gazéification.

11. Système selon la revendication 10, comprenant en outre:
- une unité (40) pour convertir le biogaz en électricité et en chaleur résiduelle; et
- une unité (50) pour convertir la chaleur résiduelle obtenue lors de la conversion du biogaz en électricité, et l'énergie thermique obtenue lors de l'incinération en électricité et en chaleur résiduelle.
